# EUROPÄISCHE PATENTSCHRIFT

(11) **EP 3 291 752 B1**
(45) Veröffentlichungstag und Bekanntmachung des Hinweises auf die Patenterteilung: **23.06.2021**
(21) Anmeldenummer: 16723687.6
(22) Anmeldetag: 04.05.2016
(51) Int. Cl.: A61B 17/72, A61F 2/42

(54) **ARTHRODESEN-IMPLANTAT**
ARTHRODESIS IMPLANT
IMPLANT D'ARTHRODÈSE

(30) Priorität: 06.05.2015 DE 102015107056
(43) Veröffentlichungstag der Anmeldung: 14.03.2018
(73) Patentinhaber: Syntellix AG, 30159 Hannover (DE)
(72) Erfinder: WINGENFELD, Carsten, 53347 Alfter (DE); SCHAVAN, Robert, 30159 Hannover (DE); SEITZ, Jan-Marten, 30159 Hannover (DE); LUCAS, Arne, 30159 Hannover (DE)
(74) Vertreter: Köllner, Malte
(86) Internationale Anmeldenummer: PCT/EP2016/060017
(87) Internationale Veröffentlichungsnummer: WO 2016/177790

(56) Entgegenhaltungen:
- WO-A1-01/17445
- WO-A1-2013/177252
- WO-A2-2010/017959
- DE-A1-102011 082 210
- US-A1- 2011 301 653
- US-A1- 2013 131 822

## Beschreibung

Bei der Erfindung handelt es sich um ein Implantat zur Osteosynthese der PIP-Arthrodese oder der DIP-Arthrodese kleiner Gelenke sowie zur Osteosynthese und Stabilisierung von Frakturen kleiner Knochen des menschlichen Skeletts, das vom menschlichen Körper resorbierbar ist. Es besteht aus zwei aneinandergesetzten Schäften. Zwischen den Schäften befindet sich mindestens eine Fixierungshilfe, die über den Durchmesser des Schafts hinausragt. Das Implantat besteht aus einem vom menschlichen Körper resorbierbaren Werkstoff auf der Basis von Magnesium. Durch die Erfindung wird ein Implantat bereitgestellt, das eine extrem hohe Zugfestigkeit, eine hervorragende Stabilität, beste Verheilungseigenschaften sowie eine optimal angepasste Resorptionsgeschwindigkeit aufweist.

Fehlstellungen von Zehen- oder Fingergelenken sind weit verbreitete Erkrankungen des menschlichen Skeletts. Sie sind teils erblich bedingt, teils erworben durch ungünstige Lebensumstände, etwa durch Tragen mangelhaften Schuhwerks, einseitige dauerhafte Belastung einzelner Gelenke wie langes Stehen auf hartem Untergrund und dergleichen. Häufige Erscheinungsbilder sind das Krankheitsbild des Hallux Valgus und die damit häufig einhergehende sogenannte Hammerzehe. Als Folge der knöchernen Missbildungen und Fehlstellungen treten starke Schmerzen der Gelenke mit Einschränkungen der Beweglichkeit auf, begleitet von Schwellungen und entzündlichen Erscheinungen. Die Betroffenen vermeiden infolgedessen sportliche Aktivitäten, dann das Laufen, Gehen oder Greifen und zuletzt oft alle Arten der Bewegung mit der fatalen Folge, dass ein Teufelskreis in Gang kommt, weil sich bei mangelnder Bewegung die Probleme weiter entwickeln und verstärken.

Eine Behandlungsmöglichkeit besteht darin, das schmerzende Zehengelenk durch einen chirurgischen Eingriff in seiner Lage und Ausrichtung zu korrigieren und anschließend zu versteifen. Eine Versteifung im Mittelgelenk (proximales Interphalangealgelenk) wird als PIP-Arthrodese, eine Versteifung im Endgelenk (distales Interphalangealgelenk) wird als DIP-Arthrodese bezeichnet. Gleiches gilt für die Fingerknochen, wobei dort überwiegend das Mittelgelenk versteift wird. Durch die Versteifung wird zwar das Gelenk fixiert, der Schmerz aber ausgeschaltet. In der Folge ist das Gelenk nicht mehr beweglich, der Fuß oder die Hand kann jedoch insgesamt wieder schmerzfrei belastet und bewegt werden und somit kehrt der Patient zu einer besseren Mobilität zurück. Beim Fuß kommen sowohl PIP- als auch DIP-Arthrodese zum Einsatz, bei der Hand in der Regel vorwiegend die DIP-Arthrodese.

Der Stand der Technik kennt hierfür in der Chirurgie der Hand und des Fußes Drähte und Stifte aus Edelstahl oder Titan, so genannte Kirschnerdrähte. Diese werden temporär und oft perkutan gelegt und nach einer gewissen Zeit der Ausheilung und Verwachsung des Gelenks in einer zweiten Operation wieder entfernt. Diese Behandlung erfordert eine zweite Operation, die mit Kosten verbunden ist und den Patienten physisch und psychisch belastet.

Im Zuge des Fortschritts der Implantat-Technologie sind daher Stifte, Schrauben und Platten entwickelt worden, die das Gelenk dauerhaft fixieren und nicht mehr entfernt werden. Diese lebenslang im Knochen verbleibenden Implantate haben diverse Vorteile. Für den Patienten bringen sie eine Erleichterung, da eine zweite Operation mit den Risiken der Narkose und der Infektion entfällt. Sie bieten auch eine Kostenersparnis, da kein erneuter Eingriff und Klinikaufenthalt nötig ist. Es werden in erster Linie sterile Nägel, Stifte, Schrauben, Platten und Schäfte aus Titan, Tantal oder Edelstahl verwendet. Die permanent liegenden Implantate haben aber auch Nachteile, die darin bestehen, dass sie sich lockern können. Beim menschlichen Organismus unterliegen die meisten Zellen einem ständigen Ab- und Wiederaufbau, dessen Intensität und Geschwindigkeit mit dem Lebensalter zusammen hängt. Auch die Knochenzellen erneuern sich. Gerade bei älteren Menschen treten aber Probleme der Knochenerneuerung auf, gemeinhin bekannt unter dem Begriff der Osteoporose. Durch hormonelle Faktoren in Verbindung mit Mineralstoffmangel können der Knochenaufbau bzw. die Knochenerneuerung gestört sein. Dann können sich anfangs fest liegende Implantate oder Prothesen lockern und zu verschiedenen Beschwerden führen, mit der Folge, dass sie eines Tages doch operativ erneuert werden müssen. Gerade das hätte aber möglichst dauerhaft vermieden werden sollen. Ein weiterer Nachteil ist, dass bei Unfällen an Knochen, die mittels solcher Implantate verbunden sind, äußerst komplizierte Splitterbrüche auftreten können.

Ein operationstechnisches Problem besteht darin, dass knochenverbindende Implantate - besonders dann, wenn ein bestimmter Krümmungswinkel eingehalten werden muss - während der Operation schwer zu fixieren sind und sich axial verschieben oder um kleine Winkel um die Längsachse rotieren können. Für den chirurgischen Eingriff stellt dies ein erhebliches Risiko dar, weil das operierte Gelenk nach der Operation exakt in jener Stellung fixiert sein soll, die physiologisch sinnvoll ist. Einen Ausweg aus dieser Problematik bieten Fixierungselemente in Form von Nasen, Punkten, Riefen, Rillen oder Schienen, die am Implantat angebracht sein können und dem Operateur helfen, das Implantat exakt axial und rotationsfest zu positionieren. An dauerhaft implantierten knochenverbindenden Pins aus Titan, Tantal oder Edelstahl können derlei Fixierungselemente aber nicht angesetzt werden, weil damit dauerhaft ein Distanzelement als Spalten zwischen den zu verbindenden Knochen wirkt, was sich destabilisierend auswirkt, den Heilungsprozess behindert und eine Knochen - oder Gelenkfusion verhindert.

Permanent liegende Implantate mit hoher Festigkeit haben darüber hinaus den weiteren Nachteil, dass ein Stress-Shielding-Effekt auftreten kann. Darunter versteht man das Problem, dass fest liegende Knochenimplantate starke Kräfte in den Knochen einleiten, wodurch es an bestimmten Stellen des Knochens zur Überlastung kommt, während gleichzeitig an anderen Stellen Kräfte vom Knochen ferngehalten werden, dieser also entlastet wird. Der Knochen benötigt aber eine ständige und gleichmäßige Stimulation mit Kräften, die im Bereich der natürlichen Knochenfestigkeit von 100 bis 200 MPa liegt, um sich zu erneuern. Fehlt die Stimulation, kommt es im Lauf der Zeit zu einem Knochenabbau und zur Lockerung. Der Stress-Shielding-Effekt hat weitreichende Folgen. Der Heilungsprozess kann sich verlangsamen und der entlastete Knochen sogar zurückbilden. Gerade dies soll aber vermieden werden, der Knochen soll aufgebaut, stabilisiert und ausgeheilt werden. WO 01/17445 A1, US 2011/0301653 A1, US 2013/0131822 A1 und WO 2013/177252 A1 offenbaren solche Implantate.

Zur Vermeidung dieser Probleme sind aus dem Stand der Technik sogenannte bioresorbierbare oder biodegradierbare Implantate aus Polyglycoliden oder Polylactiden bekannt, die im Knochen unter dem Einfluss von Gewebsflüssigkeit allmählich aufgelöst und abgebaut werden und nach der Heilung nicht operativ entfernt werden müssen. Sie werden sukzessive durch neu gebildetes körpereigenes Knochengewebe ersetzt. Die Versorgung von Zehengelenken durch Implantate, die aus Polylactiden aufgebaut sind, wird beschrieben in der Dissertationschrift "Prospektive Untersuchung interner Osteosynthesen mit resorbierbaren Implantaten in der Vorfußchirurgie" von Panagiotis Bouliopoulos, Medizinische Fakultät der Ludwig-Maximilians-Universität, München (2005). Aus den Untersuchungen ist festzuhalten, dass der Ersatz eines permanent liegenden Implantats durch ein resorbierbares, sich allmählich auflösendes Implantat erhebliche Vorteile mit sich bringt. So wird insbesondere der oben erwähnte Stress-Shielding Effekt vermieden, subjektiv empfundene Fremdkörpergefühle werden vermindert und eventuelle spätere Eingriffe aufgrund von Brüchen können leicht durchgeführt werden.

Dem stehen jedoch auch Nachteile der polymeren Werkstoffe gegenüber. Ein erster erheblicher Nachteil der Implantate auf Polylactidbasis ist deren geringe mechanische Belastbarkeit. Die Zugfestigkeit von Lactidpolymeren ist geringer als 50 MPa. Zudem sind sie thermisch wenig stabil. Im Zeitraum des Knochenaufbaus, für den je nach Konstitution des Patienten etwa 12 Wochen veranschlagt werden müssen, kommt es sehr auf die mechanische Belastbarkeit des Implantats an, die man bei polymeren Implantaten als unzureichend bezeichnen muss. Als weiterer Nachteil ist zu nennen, dass beim hydrolytischen Abbauprozess der Polymere ein saures Milieu um das Implantat entsteht. Dieses kann gewebeschädigend wirken und dazu führen, dass das Implantatlager nicht knöchern durchbaut wird. Zum Teil verbleiben über einen längeren Zeitraum Löcher im Knochen.

Anstelle resorbierbarer polymerer Werkstoffe werden Magnesiumlegierungen als resorbierbare metallische Werkstoffe bereits seit einiger Zeit in der Medizintechnik eingesetzt. So beschreibt die Patentschrift EP 0 966 979 B1 die Verwendung einer Magnesiumlegierung als Stent bzw. Gefäßwandstütze in Blutgefäßen, ohne die Zusammensetzung der Legierung näher zu nennen. In der Offenlegungsschrift DE 101 28 100 A1 werden diverse Magnesiumbasislegierungen als geeignet für Drähte, Stifte, Schrauben, Platten usw. in der Human- und Tiermedizin beschrieben. Diese Legierungen enthalten jedoch Aluminium in Massenanteilen bis zu 16 Gew.-% und sind nach heutiger Kenntnis wegen der Toxizität des Aluminiums (Nervenschädigung, Verdacht auf Auslösung der Alzheimerschen Krankheit) als biodegradierbare Implantate nicht geeignet. Die Gebrauchsmusterschrift DE 20 2008 018 U1 beschreibt ein Implantat auf Basis einer Magnesiumlegierung, die Yttrium, Seltene Erden und Zirkonium enthält, es werden jedoch keine Hinweise gegeben, wie das Implantat zur Behandlung spezifischer Krankheitsbilder ausgeführt sein muss. Es wird als Ausführungsform jedoch nur eine einfache Schraubenform offenbart. Die Offenlegungsschrift DE 10 2011 082 210 A1 beschreibt die Herstellung von biodegradierbaren Halbzeugen aus Magnesiumbasislegierungen in einem aufwändigen, mehrstufigen pulvermetallurgischen Prozess und erwähnt lediglich die Möglichkeit, daraus medizinische Implantate herzustellen. Eine nähere Beschreibung entsprechender Implantate findet jedoch nicht statt. WO 2010/017959 A2 offenbart weitere Implantate aus einer Magnesiumbasislegierung.

Aufgabe der vorliegenden Erfindung ist es deshalb, die beschriebenen Nachteile von nicht biodegradierbaren Implantaten sowie biodegradierbarer Implantate auf Lactidbasis zu vermeiden, wobei ein biodegradierbares Material auf Basis einer Magnesiumlegierung als Implantat zur Osteosynthese der PIP-Arthrodese oder der DIP-Arthrodese kleiner Gelenke sowie zur Osteosynthese und Stabilisierung von Frakturen kleiner Knochen des menschlichen Skeletts nutzbar gemacht werden soll. Gelöst wird die Aufgabe durch ein Implantat gemäß Anspruch 1 aus einem vom menschlichen Körper resorbierbaren metallischen Werkstoff auf der Basis von Magnesium.

Bei der Erfindung handelt es somit um ein resorbierbares metallisches Implantat speziell zur Arthrodese kleiner Gelenke und Knochen oder deren Osteosynthese auf Basis eines Magnesiumwerkstoffs, der sich im Körper nach Fixierung des Gelenks oder des Knochens und der Fusion der Knochen allmählich auflöst, in organische Substanzen umgebaut und durch körpereigenes Knochenmaterial ersetzt wird. Die bei der Degradation entstehenden Magnesiumionen oxidieren und werden teilweise als Oxide oder Hydroxide in den Knochen eingebaut, teilweise verstoffwechselt und über den Urin ausgeschieden. Das Implantat kann an Zehengelenken, Zehen- und Fußknochen, an Fingergelenken und bei Frakturen der Finger- und Fußknochen eingesetzt werden. Die beiden Schäfte des Implantats werden als proximaler und distaler Schaft bezeichnet, wobei der proximale Schaft derjenige Schaft ist, der zur Körpermitte hin gerichtet ist, während der distale Schaft derjenige Schaft ist, der von der Körpermitte weg gerichtet ist.

Die Schäfte des Implantats sind vorzugsweise konusförmig. Jeder Schaft weist mindestens eine Längsnut auf. Die Längsnuten erlauben einen schmalen, dem Knochen angepassten Bohrkanal, wodurch wertvolle Knochensubstanz gerade und besonders bei kleinen Knochen erhalten bleibt. Die Längsnuten wirken einer Rotation entgegen, da sie sich in das intramedulläre, etwas weichere Gewebe einprägen. Das Implantat ist somit äußerst torsionsstabil. Die Ausfräsungen vergrößern die Oberfläche, was für die anfängliche Verwachsung bzw. Verzahnung des Implantats mit dem Knochengewebe günstig ist. Durch die Modifikation der Oberfläche lässt sich zusätzlich die Resorptionsgeschwindigkeit anpassen. Es ist besonders bevorzugt, wenn die Zahl der Längsnuten zwei bis acht, vorzugsweise sechs beträgt. Angesetzt an das Implantat sind Fixierungshilfen. Diese können die Form von Flügelansätzen besitzen. Sie dienen als Anschlag beim Einführen des Implantates in die proximale Bohrung sowie beim Aufbringen des distalen Knochens auf das Implantat. Dies erlaubt dem Chirurgen eine optimale Fixierung des Implantats während der Operation und auch zu tiefes Vorbohren bleibt ohne Einschränkungen für den optimalen Sitz des Implantates. Eine Spaltenbildung und damit die Behinderung der Fusion bei der Ausheilung muss nicht befürchtet werden, da sich die dünne Fixierungshilfe rasch auflöst. Es sind zwei bis acht, ganz bevorzugt aber drei Fixierungshilfen vorhanden. Die Fixierungshilfen sind vorzugsweise in der Form von Flügelansätzen ausgeführt. Sie können jedoch auch eine beliebige andere Form haben; so können sie beispielsweise als Nasen, Schienen, Riefen oder Fixationspunkte ausgeführt sein. Die Fixierungshilfen sind vorzugsweise in gleichen Abständen um den Schaft herum angeordnet. Ihre Dicke im Querschnitt beträgt 0,10 bis 2,00 mm und sie ragen radial über den Schaft um 0,25 bis 4,00 mm hinaus. Es hat sich als vorteilhaft erwiesen, wenn die Dicke im Querschnitt der Fixierungshilfen zwischen 0,20 und 2,00 mm beträgt und die Fixierungshilfen radial über den Schaft um 0,50 bis 4,00 mm hinausragen. Außerdem hat es sich als besonders vorteilhaft erwiesen, wenn die Dicke im Querschnitt der Fixierungshilfen zwischen 0,20 und 1,00 mm beträgt und die Fixierungshilfen radial über den Schaft um 0,50 bis 2,00 mm hinausragen. In einer äußerst vorteilhaften Ausführungsform beträgt die Dicke im Querschnitt der Fixierungshilfen zwischen 0,20 und 0,80 mm und die Fixierungshilfen ragen radial über den Schaft um 0,50 bis 1,00 mm hinaus. Die Fixierungshilfen ermöglichen dem operierenden Chirurgen die exakte Positionierung des Implantats und sie lösen sich im Verlauf des Heilungsprozesses und der Resorption auf. Die Gefahr einer Spaltenbildung zwischen den miteinander verbundenen Knochen, eine Destabilisierung des operativ versorgten Gebiets und damit eine Behinderung der Fusion ist ausgeschlossen.

Das Implantat besteht aus zwei Schäften, einem distalen und einem proximalen Schaft. Diese sind miteinander verbunden. Sie können beispielsweise miteinander verschweißt oder durch einen bioabsorbierbaren Kleber verbunden sein. In einer möglichen Ausführungsvariante sind distaler und proximaler Schaft aus einem Stück gefertigt. Um eine optimale Stabilität unter Beachtung der Degradierungs- und Verbauungsgeschwindigkeit des Materials zu gewährleisten, ist darauf zu achten, dass die Länge der Schäfte jeweils 5,00 bis 75,00 mm beträgt, der Durchmesser der Schäfte 1,50 bis 20,00 mm beträgt, die Dicke der Fixierungshilfen 0,10 bis 2,00 mm beträgt und der Überstand der Fixierungshilfen über den Durchmesser der Schäfte 0,25 bis 4,00 mm beträgt. Es hat sich herausgestellt, dass die Länge der Schäfte vorzugsweise jeweils 5,00 bis 15,00 mm beträgt, der Durchmesser der Schäfte vorzugsweise 2,00 bis 10,00 mm beträgt, die Dicke der Fixierungshilfen vorzugsweise 0,20 bis 1,00 mm beträgt und der Überstand der Fixierungshilfen über den Durchmesser der Schäfte vorzugsweise 0,50 bis 2,00 mm beträgt. Eine ganz besonders vorteilhafte Ausführungsform ist dadurch gekennzeichnet, dass die Länge des proximalen Schafts 10,00 bis 15,00 mm beträgt, die Länge des distalen Schafts 5,00 bis 11,00 mm beträgt und der Durchmesser der beiden Schäfte jeweils 2,40 bis 3,60 mm beträgt.

Abgewinkelte Implantate sind aus anatomischen Gründen zu bevorzugen, da sie einer physiologischen Standard-Stellung des überbrückten und damit deaktivierten Gelenks am ehesten entsprechen. Der Winkel der zwischen den Schäften aufgespannt wird, liegt zwischen 155 und 175 Grad, vorzugsweise zwischen 160 und 175 Grad und er beträgt ganz bevorzugt ungefähr 160 Grad. Bevorzugt ist einer der Schäfte zusätzlich mit Quernuten versehen. Die Quernuten dienen insbesondere im intraoperativen Handling zur axialen Stabilisierung nach dem Einbringen des proximalen Anteils des Implantates in den proximalen Knochen. Im etwas flexiblen bzw. elastischen intramedullären Gewebe wird ansatzweise ein Formschluss im Implantatlager erzeugt. Nachdem das Implantat gegen die Fixierungshilfen eingebracht wurde, verhindert dieser Formschluss, dass das Implantat unbeabsichtigt wieder aus dem Implantatlager herausgezogen wird. Auf der distalen Seite des Implantates sollen bei dieser möglichen Ausführungsform keine Quernuten eingebracht werden. Ein Vorteil hiervon ist die Erhöhung der Sicherheit in der OP-Technik. Immer wieder kommt es vor, dass Implantate falsch herum gesetzt werden. Durch die Quernuten ist es gewährleistet, dass der anwendende Mediziner zwischen distalem und proximalem Schaft unterscheiden kann. In einer alternativen Ausführungsform der Erfindung sind beide Schäfte mit Quernuten versehen, sodass auch zwischen distalem Schaft und Knochen ein besonderer Halt entsteht.

Das Implantat ist dadurch gekennzeichnet, dass als resorbierbarer Werkstoff eine Magnesiumlegierung aus dem Legierungssystem MgYREZr eingesetzt wird, das heißt die Legierung umfasst zumindest Magnesium, Zirkonium, Yttrium sowie weitere Seltenerdmetalle.

In einer möglichen Ausführungsform enthält die Magnesiumbasislegierung 0,10 bis 1,00 Gew.-% Zirkonium, 4,75 bis 5,00 Gew.-% Yttrium und 2,50 bis 4,00 Gew.-% weitere Seltenerdmetalle. In einer weiteren möglichen Ausführungsform enthält die Magnesiumlegierung 0,10 bis 1,00 Gew.-% Zirkonium, 3,70 bis 4,30 Gew.-% Yttrium und 2,50 bis 4,40 Gew.-% weitere Seltenerdmetalle. Entsprechende Legierungen haben eine Zugfestigkeit von 200 bis 250 MPa. Die Herstellung dieser Legierungen kann gemäß DIN EN 1753 erfolgen. Es ist ganz besonders vorteilhaft, wenn die Legierungen den für die Werkstoffe EN-MC95310 bzw. EN-MC95320 in DIN EN 1753 genannten Parametern genügen.

Erfindungsgemäß enthält die Magnesiumlegierung 0,10 bis 2,50 Gew.-% Zirkonium, 0,01 bis 0,80 Gew.-% Zink, 1,50 bis 5,00 Gew.-% Yttrium und 2,50 bis 5,00 Gew.-% Seltenerdmetalle,. Hierdurch kann eine erhöhte Zugfestigkeit von bis zu 300 MPa erreicht werden.

Damit liegt die Zugfestigkeit der aufgeführten Magnesiumlegierungen deutlich über der Festigkeit der Lactidpolymere von max. ca. 50 MPa. Ihre Zugfestigkeit liegt vielmehr nahe bei den Zugfestigkeiten und Elastizitätswerten, die an menschlichen Knochen gemessen werden. Als Besonderheit der vorliegenden Erfindung ist zudem festzuhalten, dass alle Legierungen so hergestellt oder gereinigt sein müssen, dass der Gehalt an Kupfer, Aluminium, Nickel und Eisen jeweils unterhalb von 0,01 Gew.-% in der fertigen Legierung liegt, damit toxische Erscheinungen und eine Beeinträchtigung der Korrosionseigenschaften sicher zu vermeiden sind.

Im Gegensatz zur toxischen Wirkung durch Verunreinigungen haben die bei der Auflösung frei werdenden Magnesiumionen verschiedene positive physiologische Effekte im Körper des Patienten. Da die Magnesiumionen während der Phase der Auflösung im Körper zu einer Stabilisierung des Elektrolythaushalts beitragen, üben sie eine positive Wirkung auf das Knochenwachstum aus und lösen durch Einflüsse auf das Nervengewebe im operierten Gebiet weitere verschiedene positive Heilungswirkungen aus. Das Magnesium wird über die Nieren ausgeschieden. Von Intoxikationen durch einen erhöhten Magnesiumspiegel ist noch nie berichtet worden.

Der große Vorteil bei Verwendung der erfindungsgemäßen biodegradierbaren Magnesiumimplantate besteht darin, dass sowohl eine zweite Operation zur Entfernung des Implantats als auch die Notwendigkeit einer späteren zweiten Operation wegen Lockerung des Implantats ausgeschlossen ist, da es im Körper vollständig abgebaut wird. Als weiterer Vorteil ist zu nennen, dass magnesiumbasierte bioresorbierbare Implantate gegenüber den polymeren, lactidbasierten Implantaten eine erheblich höhere Festigkeit aufweisen. In der ungefähr zwölf Wochen dauernden Phase der Ausheilung nach der Operation, während der das Implantat noch den Großteil der auftretenden Kräfte aufnehmen muss, ist eine optimale Stabilisierung des versteiften Gelenks gegeben, da die Festigkeit der Magnesiumlegierung deutlich über der von polymeren Implantatwerkstoffen liegt.

Es ist außerdem vorteilhaft, dass die Degradation des Magnesiumimplantats so langsam verläuft, dass der Abbau des Implantats in einer Geschwindigkeit verläuft, die der Aufbaurate des Knochens angepasst ist. Das Knochenwachstum an der operierten Stelle und die Degradation des Implantats befinden sich in einem Gleichgewicht. Das neu gebildete Knochengewebe übernimmt dabei sukzessive die auftretenden Kräfte. Der Stress-Shielding-Effekt wird vermieden. Über die Komposition der Legierung kann zudem Einfluss auf die Kinetik der Degradation genommen werden und eine Anpassung an die physiologischen Gegebenheiten bestimmter Indikationen oder Patientengruppen erfolgen.

Die Erfindung wird durch die beigelegten Zeichnungen erläutert.
Fig. 1 zeigt eine erste Ausführungsform der Erfindung in einer Seitenansicht.
Fig. 2 zeigt die erste Ausführungsform der Erfindung in einer Querschnittsansicht.
Fig. 3 zeigt den proximalen Schaft (1) der ersten Ausführungsform in einer Längsschnittansicht.
Fig. 4 zeigt den distalen Schaft (2) der ersten Ausführungsform der Erfindung in einer Längsschnittansicht.
Fig. 5 zeigt eine zweite, nicht erfindungsgemäße Form des Implantats in einer Seitenansicht.
Fig. 6 zeigt eine dritte Ausführungsform der Erfindung in einer Seitenansicht.

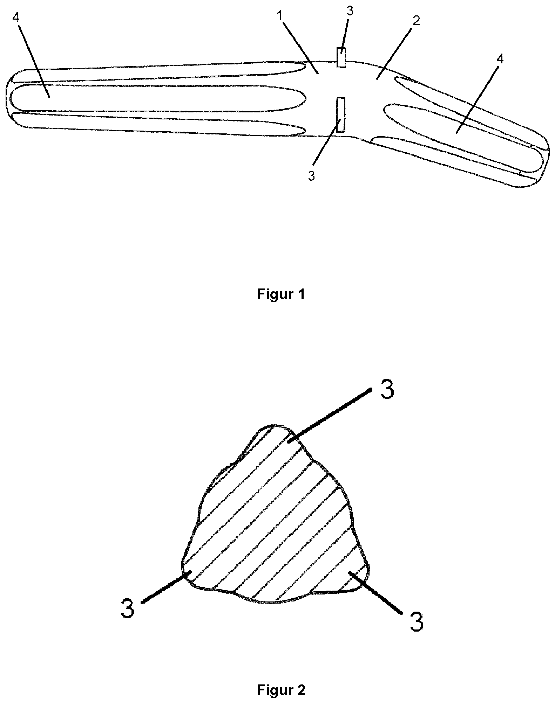

Fig. 1 zeigt eine erste Ausführungsform der Erfindung in einer Seitenansicht. Die zur Herstellung verwendete Magnesiumlegierung enthält 0,10 bis 2,50 Gew.-% Zirkonium, 0,01 bis 0,80 Gew.-% Zink, 1,50 bis 5,00 Gew.-% Yttrium und 2,50 bis 5 Gew.-% weitere Seltenerdmetalle. Sie besteht aus einem proximalen Schaft (1) sowie aus einem distalen Schaft (2). Die Länge des proximalen Schafts (1) beträgt 12,40 Millimeter und die Länge des distalen Schafts (2) beträgt 6,20 mm. Der Winkel zwischen proximalem Schaft (1) und distalem Schaft (2) beträgt 160 Grad. Dieser Winkel ist den physiologischen Gegebenheiten des Patienten anzupassen und kann deshalb in anderen Ausführungsformen entsprechend abweichen. Zwischen proximalem Schaft (1) und distalem Schaft (2) befinden sich drei Flügelansätze als Fixierungshilfen (3). Diese besitzen eine Dicke von 0,30 mm und einen Überstand von 1,00 mm. Die Fixierungshilfen (3) lösen sich nach der Operation rasch auf, so dass eine Spaltenbildung zwischen den durch proximalen Schaft (1) und distalen Schaft (2) verbundenen Knochen vermieden wird. Die Schäfte (1, 2) sind mit jeweils sechs Längsnuten (4) versehen, die eine Vergrößerung der Oberfläche des Implantats bewirken. Dies beeinflusst wiederum die Resorptionsgeschwindigkeit des Implantats. Darüber hinaus verhindern die Längsnuten (4) ein Verdrehen des Implantats im Knochen. Durch die Kombination der erfindungsgemäßen Magnesiumlegierung und des speziellen Aufbaus ergibt sich ein Implantat, das eine extrem hohe Zugfestigkeit von bis zu 300 MPa, eine hervorragende Stabilität, beste Verheilungseigenschaften sowie eine optimal angepasste Resorptionsgeschwindigkeit aufweist.

Fig. 2 zeigt den proximalen Schaft (1) der ersten Ausführungsform in einer Querschnittsansicht. Hierbei werden auch die drei Fixierungshilfen (3) gezeigt, die in gleichen Abständen um den proximalen Schaft (1) angeordnet sind.

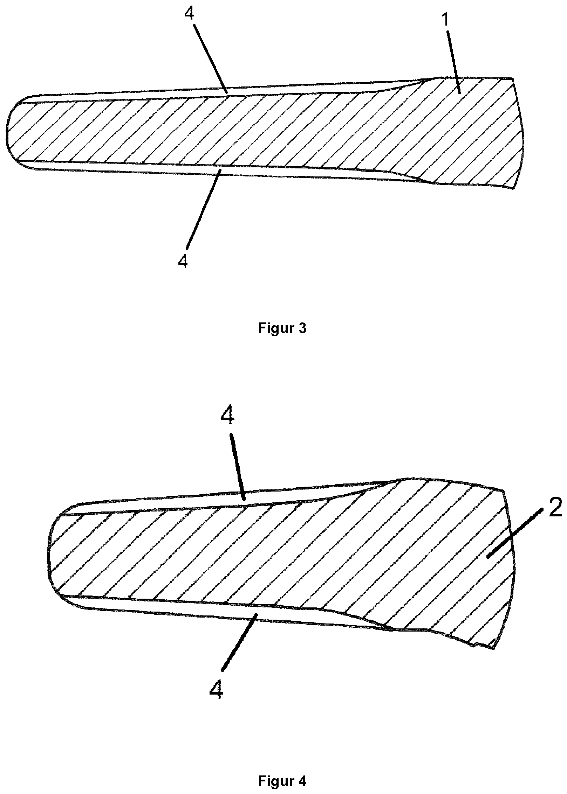

Fig. 3 zeigt den proximalen Schaft (1) in einer Querschnittsansicht. Das Profil der Längsnut (4) kann mit einem Radiusfräser erzeugt werden.

Fig. 4 zeigt den distalen Schaft (2) in einer Querschnittsansicht. Auch der distale Schaft (2) ist mit Längsnuten (4) versehen.

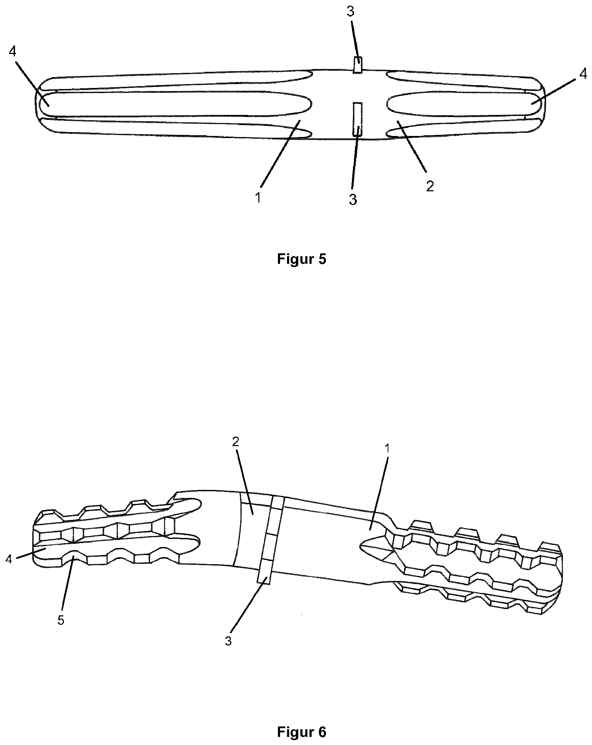

Fig. 5 zeigt eine zweite, nicht erfindungsgemäße Form des Implantats. Die zur Herstellung verwendete Magnesiumlegierung enthält 0,10 bis 2,50 Gew.-% Zirkonium, 0,01 bis 0,80 Gew.-% Zink, 1,50 bis 5,00 Gew.-% Yttrium und 2,50 bis 5,00 Gew.-% weitere Seltenerdmetalle. Zwischen proximalem Schaft (1) und distalem Schaft (2) besteht hierbei ein Winkel von 180 Grad. Ansonsten entspricht diese Ausführungsform hinsichtlich ihrer Abmessungen der ersten Ausführungsform.

Fig. 6 zeigt eine dritte Ausführungsform der Erfindung. Die zur Herstellung verwendete Magnesiumlegierung enthält 0,10 bis 2,50 Gew.-% Zirkonium, 0,01 bis 0,80 Gew.-% Zink, 1,50 bis 5,00 Gew.-% Yttrium und 2,50 bis 5,00 Gew.-% weitere Seltenerdmetalle. Proximaler Schaft (1) und distaler Schaft (2) verfügen hierbei sowohl über Längsnuten (4) als auch Quernuten (5). Die Quernuten (5) verleihen dem Implantat einen verbesserten Halt im Knochen.

### Bezugszeichenliste

1. Proximaler Schaft
2. Distaler Schaft
3. Fixierungshilfe
4. Längsnut
5. Quernut

## Patentansprüche

1. Implantat zur Osteosynthese der PIP-Arthrodese oder der DIP-Arthrodese kleiner Gelenke sowie zur Osteosynthese und Stabilisierung von Frakturen kleiner Knochen des menschlichen Skeletts, wobei das Implantat
(a) aus einem proximalen Schaft (1) und einem distalen Schaft (2) besteht, die unter einem Winkel von 155 bis 175 Grad miteinander verbunden sind,
wobei jeder Schaft (1, 2) mindestens eine Längsnut (4) aufweist;
(b) zwischen den Schäften (1, 2) mindestens eine Fixierungshilfe (3) vorhanden ist, die über den Durchmesser des Schafts (1, 2) hinausragt;
(c) die Länge der Schäfte (1, 2) jeweils 5,00 bis 75,00 mm beträgt, der Durchmesser der Schäfte (1, 2) 1,50 bis 20,00 mm beträgt, die Dicke der Fixierungshilfen (3) 0,10 bis 2,00 mm beträgt und der Überstand der Fixierungshilfen (3) über den Durchmesser der Schäfte (1, 2) 0,25 bis 4,00 mm betragt;
(d) das Implantat aus einer vom menschlichen Körper resorbierbaren Magnesiumbasislegierung besteht, die 0,10 bis 2,50 Gew.-% Zirkonium, 0,01 bis 0,80 Gew.-% Zink, 1,50 bis 5,00 Gew.-% Yttrium und 2,50 bis 5,00 Gew.-% sonstige Seltenerdmetalle enthält; und
(e) der Gehalt an Kupfer, Aluminium, Nickel und Eisen jeweils unter 0,01 Gew.-% beträgt.

2. Implantat nach Anspruch 1, **dadurch gekennzeichnet, dass** die Zahl der Längsnuten (4) zwei bis acht, vorzugsweise sechs, beträgt.

3. Implantat nach einem der vorhergehenden Ansprüche, **dadurch gekennzeichnet, dass** an den Schäften (1, 2) zwei bis acht, vorzugsweise drei Fixierungshilfen (3) vorhanden sind, die symmetrisch um die Schäfte (1, 2) herum angeordnet sind.

4. Implantat nach Anspruch 3, **dadurch gekennzeichnet, dass** die Dicke im Querschnitt der Fixierungshilfen (3) zwischen 0,20 und 2,00 mm beträgt und die Fixierungshilfen (3) radial über die Schäfte (1, 2) um 0,50 bis 4,00 mm hinausragen.

5. Implantat nach Anspruch 4, **dadurch gekennzeichnet, dass** die Dicke im Querschnitt der Fixierungshilfen (3) zwischen 0,20 und 1,00 mm beträgt und die Fixierungshilfen (3) radial über die Schäfte (1, 2) um 0,50 bis 2,00 mm hinausragen.

6. Implantat nach Anspruch 4, **dadurch gekennzeichnet, dass** die Dicke im Querschnitt der Fixierungshilfen (3) zwischen 0,20 und 0,80 mm beträgt und die Fixierungshilfen (3) radial über die Schäfte (1, 2) um 0,50 bis 1,00 mm hinausragen.

7. Implantat nach einem der Ansprüche 1-5, **dadurch gekennzeichnet, dass** die Länge der Schäfte (1, 2) jeweils 5,00 bis 15,00 mm beträgt, der Durchmesser der Schäfte (1, 2) 2,00 bis 10,00 mm beträgt, die Dicke der Fixierungshilfen (3) 0,20 bis 1,00 mm beträgt und der Überstand der Fixierungshilfen (3) über den Durchmesser der Schäfte (1, 2) 0,50 bis 2,00 mm beträgt.

8. Implantat nach einem der vorhergehenden Ansprüche, **dadurch gekennzeichnet, dass** die Länge des proximalen Schafts (1) 10,00 bis 15,00 mm beträgt, die Länge des distalen Schafts (2) 5,00 bis 11,00 mm beträgt und der Durchmesser der beiden Schäfte (1, 2) jeweils 2,40 bis 3,60 mm beträgt.

9. Implantat nach einem der vorhergehenden Ansprüche, **dadurch gekennzeichnet, dass** der Winkel zwischen den Schäften (1, 2) zwischen 155 und 160 Grad beträgt.

10. Implantat nach einem der vorhergehenden Ansprüche, **dadurch gekennzeichnet, dass** der Winkel zwischen den Schäften (1, 2) ungefähr 160 Grad beträgt.

11. Implantat nach einem der vorhergehenden Ansprüche, **dadurch gekennzeichnet, dass** ein oder beide Schäfte (1, 2) Quernuten (5) aufweisen.

12. Implantat nach Anspruch 1, **dadurch gekennzeichnet, dass** die Magnesiumbasislegierung, 0,10 bis 1,00 Gew.-% Zirkonium, 4,75 bis 5,00 Gew.-% Yttrium und 2,50 bis 4,00 Gew.-% sonstige Seltenerdmetalle enthält.

13. Implantat nach Anspruch 1, **dadurch gekennzeichnet, dass** die Magnesiumbasislegierung 0,10 bis 1,00 Gew.-% Zirkonium, 3,70 bis 4,30 Gew.-% Yttrium und 2,50 bis 4,40 Gew.-% sonstige Seltenerdmetalle enthält.

## Claims

1. Implant for osteosynthesis of PIP arthrodesis or DIP arthrodesis of small joints and for osteosynthesis and stabilization of fractures of small bones of the human skeleton, wherein
(a) the implant consists of a proximal shaft (1) and a distal shaft (2) which are connected to each other at an angle of 155 to 175 degrees, each shaft (1, 2) having at least one longitudinal groove (4);
(b) between the shafts (1, 2) there is at least one fixing aid (3) which protrudes beyond the diameter of shaft (1, 2);
(c) the length of each shaft (1, 2) is 5.00 to 75.00 mm, the diameter of the shafts (1, 2) is 1.50 to 20.00 mm, the thickness of the fixing aids (3) is 0.10 to 2.00 mm and the protrusion of fixing aids (3) beyond the diameter of the shafts (1, 2) is 0.25 to 4.00 mm;
(d) the implant is made of a magnesium-based alloy resorbable by the human body and containing 0.10 to 2.50% by weight of zirconium, 0.01 to 0.80% by weight of zinc, 1.50 to 5.00% by weight of yttrium and 2.50 to 5.00% by weight of other rare earth metals; and
(e) the copper, aluminum, nickel and iron content is each less than 0.01% by weight.

2. Implant according to claim 1, **characterized in that** the number of longitudinal grooves (4) is two to eight, preferably six.

3. Implant according to one of the preceding claims, **characterized in that** two to eight, preferably three, fixing aids (3) are present on shafts (1, 2) and are arranged symmetrically around the shafts (1, 2).

4. Implant according to claim 3, **characterized in that** the cross-sectional thickness of fixing aids (3) is between 0.20 and 2.00 mm and the fixing aids (3) protrude radially beyond the shafts (1, 2) by 0.50 to 4.00 mm.

5. Implant according to claim 4, **characterized in that** the cross-sectional thickness of fixing aids (3) is between 0.20 and 1.00 mm and the fixing aids (3) protrude radially beyond the shafts (1, 2) by 0.50 to 2.00 mm.

6. Implant according to claim 4, **characterized in that** the cross-sectional thickness of fixing aids (3) is between 0.20 and 0.80 mm and the fixing aids (3) project radially beyond the shafts (1, 2) by 0.50 to 1.00 mm.

7. Implant according to any of claims 1-5, **characterized in that** the length of each shaft (1, 2) is 5.00 to 15.00 mm, the diameter of shafts (1, 2) is 2.00 to 10.00 mm, the thickness of fixing aids (3) is 0.20 to 1.00 mm, and the protrusion of fixing aids (3) beyond the diameter of shafts (1, 2) is 0.50 to 2.00 mm.

8. Implant according to one of the preceding claims, **characterized in that** the length of proximal shaft (1) is 10.00 to 15.00 mm, the length of distal shaft (2) is 5.00 to 11.00 mm, and the diameter of shafts (1, 2) is each 2.40 to 3.60 mm, respectively.

9. Implant according to any of the preceding claims, **characterized in that** the angle between shafts (1, 2) is between 155 and 160 degrees.

10. Implant according to any of the preceding claims, **characterized in that** the angle between shafts (1, 2) is approximately 160 degrees.

11. Implant according to one of the preceding claims, **characterized in that** one or both shafts (1, 2) have transverse grooves (5).

12. Implant according to claim 1, **characterized in that** the magnesium-based alloy contains, 0.10 to 1.00 wt.% zirconium, 4.75 to 5.00 wt.% yttrium and 2.50 to 4.00 wt.% other rare earth metals.

13. Implant according to claim 1, **characterized in that** the magnesium-based alloy contains 0.10 to 1.00 wt.% zirconium, 3.70 to 4.30 wt.% yttrium and 2.50 to 4.40 wt.% other rare earth metals.

## Revendications

1. Implant pour l'ostéosynthèse de l'arthrodèse PIP ou de l'arthrodèse DIP de petites articulations et pour l'ostéosynthèse et la stabilisation de fractures de petits os du squelette humain, dans lequel l'implant
(a) consiste en une tige proximale (1) et une tige distale (2) qui sont reliées l'une à l'autre selon un angle de 155 à 175 degrés,
chaque tige (1, 2) ayant au moins une rainure longitudinale (4) ;
(b) entre les tiges (1, 2) se trouve au moins un auxiliaire de fixation (3) qui dépasse le diamètre de la tige (1, 2) ;
(c) la longueur des tiges (1, 2) est respectivement de 5,00 à 75,00 mm, le diamètre des tiges (1, 2) est de 1,50 à 20,00 mm, l'épaisseur des auxiliaires de fixation (3) est de 0,10 à 2,00 mm et la saillie des auxiliaires de fixation (3) au-delà du diamètre des tiges (1, 2) est de 0,25 à 4,00 mm ;
(d) l'implant est constitué d'un alliage à base de magnésium résorbable par le corps humain et contenant de 0,10 à 2,50 % en poids de zirconium, de 0,01 à 0,80 % en poids de zinc, de 1,50 à 5,00 % en poids d'yttrium et de 2,50 à 5,00 % en poids d'autres métaux des terres rares ; et
(e) la teneur en chacun des éléments suivants : cuivre, aluminium, nickel et fer est inférieure à 0,01 % en poids.

2. Implant selon la revendication 1, **caractérisé en ce que** le nombre de rainures longitudinales (4) est de deux à huit, de préférence six.

3. Implant selon l'une quelconque des revendications précédentes, **caractérisé en ce que** deux à huit, de préférence trois, auxiliaires de fixation (3) sont présents sur les tiges (1, 2) et sont disposés symétriquement autour des tiges (1, 2).

4. Implant selon la revendication 3, **caractérisé en ce que** l'épaisseur en section transversale des auxiliaires de fixation (3) est comprise entre 0,20 et 2,00 mm et les auxiliaires de fixation (3) dépassent radialement des tiges (1, 2) de 0,50 à 4,00 mm.

5. Implant selon la revendication 4, **caractérisé en ce que** l'épaisseur en section transversale des auxiliaires de fixation (3) est comprise entre 0,20 et 1,00 mm et les auxiliaires de fixation (3) dépassent radialement des tiges (1, 2) de 0,50 à 2,00 mm.

6. Implant selon la revendication 4, **caractérisé en ce que** l'épaisseur en section transversale des auxiliaires de fixation (3) est comprise entre 0,20 et 0,80 mm et les auxiliaires de fixation (3) dépassent radialement des tiges (1, 2) de 0,50 à 1,00 mm.

7. Implant selon l'une des revendications précédentes, **caractérisé en ce que** la longueur des tiges (1, 2) est respectivement de 5,00 à 15,00 mm, le diamètre des tiges (1, 2) est de 2,00 à 10,00 mm, l'épaisseur des auxiliaires de fixation (3) est de 0,20 à 1,00 mm et la saillie des auxiliaires de fixation (3) au-delà du diamètre des tiges (1, 2) est de 0,50 à 2,00 mm.

8. Implant selon l'une des revendications précédentes, **caractérisé en ce que** la longueur de la tige proximale (1) est de 10,00 à 15,00 mm, la longueur de la tige distale (2) est de 5,00 à 11,00 mm et le diamètre des deux tiges (1, 2) est respectivement de 2,40 à 3,60 mm.

9. Implant selon l'une quelconque des revendications précédentes, **caractérisé en ce que** l'angle entre les tiges (1, 2) est compris entre 155 et 160 degrés.

10. Implant selon l'une quelconque des revendications précédentes, **caractérisé en ce que** l'angle entre les tiges (1, 2) est d'environ 160 degrés.

11. Implant selon l'une quelconque des revendications précédentes, **caractérisé en ce qu'**une ou les deux tiges (1, 2) comprennent des rainures transversales (5).

12. Implant selon la revendication 1, **caractérisé en ce que** l'alliage à base de magnésium contient 0,10 à 1,00 % en poids de zirconium, 4,75 à 5,00 % en poids d'yttrium et 2,50 à 4,00 % en poids d'autres métaux des terres rares.

13. Implant selon la revendication 1, **caractérisé en ce que** l'alliage à base de magnésium contient 0,10 à 1,00 % en poids de zirconium, 3,70 à 4,30 % en poids d'yttrium et 2,50 à 4,40 % en poids d'autres métaux des terres rares.
